(19) Europäisches Patentamt
European Patent Office
Office européen des brevets

(11) **EP 4 008 312 A1**

(12) **EUROPEAN PATENT APPLICATION**

(43) Date of publication:
08.06.2022 Bulletin 2022/23

(21) Application number: 20306476.1

(22) Date of filing: 01.12.2020

(51) International Patent Classification (IPC):
$A61K\ 9/107^{(2006.01)}$      $A61K\ 47/32^{(2006.01)}$
$A61K\ 47/36^{(2006.01)}$      $A61K\ 47/38^{(2006.01)}$
$A61K\ 47/34^{(2017.01)}$      $A61K\ 8/06^{(2006.01)}$

(52) Cooperative Patent Classification (CPC):
A61K 9/1075; A61K 47/32; A61K 47/34;
A61K 47/36; A61K 47/38

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Calyxia
94380 Bonneuil-sur-Marne (FR)

(72) Inventors:
• LAVIELLE, Nicolas
94380 Bonneuil-sur-Marne (FR)
• DEMOULIN, Damien
94380 Bonneuil-sur-Marne (FR)

(74) Representative: Icosa
83 avenue Denfert-Rochereau
75014 Paris (FR)

(54) **EMULSION COMPRISING SIZE-CONTROLLED DROPLETS**

(57) The present invention relates to an emulsion comprising size-controlled droplets dispersed in a viscoelastic continuous phase comprising a polymer in solution in a solvent, wherein the viscoelastic continuous phase presents a ratio between the viscous modulus (G") and the elastic modulus (G') ranging from 0.2 to 10 and wherein the mean diameter of the droplets is proportional to the G'/G" ratio with 1.5 as exponent.

The invention also relates to a process for the fabrication of size-controlled droplets in a viscoelastic continuous phase of an emulsion. The invention still relates to the use of the emulsion in applications requiring size-controlled droplets.

EP 4 008 312 A1

Processed by Luminess, 75001 PARIS (FR)

**Description**

**FIELD OF INVENTION**

The present invention relates to an emulsion comprising size-controlled droplets dispersed in a viscoelastic continuous phase.

The invention also relates to a process for the fabrication of size-controlled droplets in a viscoelastic continuous phase of an emulsion.

The invention also relates to the use of the emulsion in applications requiring size-controlled droplets.

**BACKGROUND OF INVENTION**

Emulsions are complex systems used in many industrial applications where immiscible ingredients need to be blended in a stable manner. The understanding of the mechanism of formation of the droplets during emulsion formation is essential to control the process and the size of the fabricated droplets.

One can identify two phases in an emulsion: the continuous phase and the dispersed phase. The continuous phase is the medium in which the formation of droplets take place during the emulsification and the dispersed phase is composed of droplets dispersed in the continuous phase, fabricated under shear generated by the continuous phase.

The mechanism and the scaling law of formation of droplets have been extensively studied under simple shear in laminar and turbulent flows in purely viscous continuous phases, i.e. phases that have a Newtonian behavior under shear and exhibit no elastic properties. Under shear, when the viscous pressure overcomes the Laplace pressure of the droplet, the droplet is submitted to the mechanism of elongation-fragmentation, breaking down the "mother" droplet into smaller "daughter" droplets. The size of the formed droplets varies linearly with the ratio of the Laplace pressure over the viscous pressure. Emulsions in viscoelastic continuous phases have also been studied for emulsions comprising one or more surfactants. The state-of-the-art mentions that the viscosity of the continuous phase ($\eta$) needs to be higher than the viscosity of the dispersed phase ($\eta_i$) at low shear ($\gamma. < 100$ s$^{-1}$), preferentially having $0.01 < \eta_i/\eta < 1$ in order to homogeneously rupture mother droplets into daughter droplets. It is also known that the mean diameter (D) of the fabricated droplets can be calculated using the following scaling law:

$$D \sim (\eta_i/\eta)^{0.2}$$

WO 97/38787 discloses that monodisperse droplets are formed when a secondary emulsion is manufactured from a polydisperse viscoelastic primary emulsion in an aqueous continuous phase comprising from 20% to 40% by weight of surfactant, if the ratio between the viscous modulus (G") and the elastic modulus (G') verifies:

$$G''/G' < 100$$

However, these conditions cannot be extended to any viscoelastic continuous phase and in particular to viscoelastic continuous phase without surfactant.

Indeed, emulsions in industrial products usually contain large quantities of surfactants. Surfactants are very useful during fabrication and storage to facilitate droplet formation and prevent emulsion destabilisation. However, surfactants do not contribute to the product performance during use and sometimes interact negatively with other ingredients of the product formulation. Surfactants can also cause allergic reactions in applications requiring contact with the skin. Finally, surfactants can be very costly.

There is therefore a need for improved conditions for the formation of size-controlled droplets, that are compatible with a wide range of viscoelastic phases, and in particular to viscoelastic continuous phases not comprising a surfactant.

It was found by the Applicant that it is insufficient to have G"/G' < 100 to obtain homogeneous droplet fragmentation and form size-controlled, monodisperse droplets in viscoelastic media.

The purpose of the present invention is therefore to provide conditions that apply to a broader range of viscoelastic continuous phases.

It was surprisingly found that the continuous phase should preferably belong to the predominant viscous regime, having G" > G', in order to fabricate monodisperse, size-controlled droplets. A novel relation between the ratio G"/G' and the size and size-control of the emulsified droplets was found.

## SUMMARY

This invention thus relates to an emulsion comprising droplets dispersed in a viscoelastic continuous phase, said droplets having a mean diameter inferior to 100 $\mu$m,

wherein said droplets are size-controlled,
said viscoelastic continuous phase comprising at least one polymer in solution in at least one solvent,

wherein said at least one polymer is selected from cellulose derivatives, polyacrylates, polyacrylamides, polysaccharides, protein derivatives, silicone derivatives and a mixture thereof,

provided that said at least one polymer is not an alginate,
wherein said viscoelastic continuous phase present a ratio between the viscous modulus **(G")** and the elastic modulus **(G')** ranging from 0.2 to 10; and
wherein the mean diameter of said droplets is proportional to the **G'/G"** ratio with 1.5 as exponent.

According to one embodiment, said viscoelastic continuous phase does not comprise a surfactant.

According to one embodiment, said viscoelastic continuous phase comprises exactly one polymer in solution in at least one solvent;

wherein the product $\eta_{(1\%)}$.**C** ranges from 100 mPa.s to 100 000 mPa.s, preferably from 100 mPa.s to 1000 mPa.s;
wherein $\eta_{(1\%)}$ represents the viscosity of the polymer solution at 1% concentration by weight in respect to the total weight of the viscoelastic continuous phase and **C** represents the concentration of the polymer solution; and
wherein the **G"/G'** ratio is proportional to the product $\eta_{(1\%)}$.**C** with -0.3 as exponent.

According to one embodiment, said at least one polymer is in solution in at least one solvent at a concentration ranging from 1 to 20 % by weight in respect to the total weight of the viscoelastic continuous phase.

According to one embodiment, said polymer is selected from chitosan, carboxymethyl cellulose, methyl cellulose, guar, konjac, polyacrylic acid, polyethylene oxide and a mixture thereof.

According to one embodiment, said droplets are size-controlled monodisperse droplets, and wherein said **G"/G'** ratio is ranging from 1 to 5, preferably said **G"/G'** ratio is ranging from 1 to 3.

According to one embodiment, said droplets have a mean diameter ranging from 1 to 70 $\mu$m, preferably ranging from 5 to 20 $\mu$m.

According to one embodiment, said viscoelastic continuous phase further comprises at least one Newtonian fluid.

According to one embodiment, the dispersed phase comprises at least one crosslinkable material and optionally at least one initiator.

According to one embodiment, the dispersed phase further comprises at least one non-miscible active agent.

The invention also relates to a process for the manufacture of an emulsion according to the invention, comprising a step of submitting the dispersed droplets to an elongation-fragmentation mechanism in the viscoelastic continuous phase.

According to one embodiment, a shear rate ranging from 1 to 20000 s$^{-1}$ is applied during the elongation-fragmentation step, preferably from 1 to 1000 s$^{-1}$.

According to one embodiment, the process further comprises a step of addition of a Newtonian fluid into the viscoelastic continuous phase.

The invention also relates to a process for the fabrication of microcapsules comprising the steps of:

- manufacturing an emulsion as described herein above, and
- cross-linking the formed droplets.

According to one embodiment, the cross-linking of the formed droplets is performed by photopolymerization of said emulsion.

## DEFINITIONS

In the present invention, the following terms have the following meanings:

The term **"crosslinkable material"** refers to a material with the ability to form covalent bonds or relatively short sequences of covalent bonds in order to join two molecules together. According to one embodiment, the crosslinkable material is selected from at least one monomer or polymer or cross-linking agent and a mixture thereof.

According to the invention, the monomer or polymer is selected from monomers and polymers comprising at least one reactive function selected from the group consisting of acrylate, methacrylate, vinyl ether, N-vinyl ether, mercaptoester, thiolene, siloxane, epoxy, oxetane, urethane, isocyanate and peroxide functions. According to one embodiment, the monomer or polymer is selected from monomers and polymers carrying at least one of the above-mentioned reactive functions and further carrying at least one function selected from the group consisting of primary, secondary and tertiary alkylamine functions, quaternary amino functions, sulphate, sulphonate, phosphate, phosphonate, carboxylate, hydroxyl, halogen and a mixture thereof.

According to one embodiment, the monomer or polymer is selected from polyethers, polyesters, polyurethanes, polyanhydride, polycarbonate, polyureas, polyethylene glycols, polypropylene glycols, polyamides, polyacetals, polyimides, polyolefins, polysulfides, polydimethylsiloxanes, polysaccharides and a mixture thereof.

The term **"cross-linking agent"** refers to a compound carrying at least two reactive functions capable of cross-linking a monomer or a polymer, or a mixture thereof. According to the invention the at least one cross-linking agent carries at least two reactive functions selected from acrylate, methacrylate, vinyl ether, N-vinyl ether, mercaptoester, thiolene, siloxane, epoxy, oxetane, urethane, isocyanate and peroxide functions.

The term **"emulsion"** refers to a colloidal system made of two non-miscible elements, for example oil and water, which by means of specific operations manage to have a macroscopically homogeneous, but microscopically heterogeneous appearance. A first phase being the dispersed phase is present in the form of droplets dispersed in a second homogeneous phase being the continuous phase. The mean diameter of the droplets can range from 10 nm to 5 mm, preferably from 100 nm to 100 $\mu$m and most preferably from 1 $\mu$m to 70 $\mu$m.

According to a preferred embodiment, the emulsion is a single emulsion, i.e. an emulsion in which the dispersed phase is a homogeneous medium. According to one embodiment, the emulsion is a double emulsion, i.e. an emulsion in which the dispersed phase droplets are themselves composed a solid or liquid phase dispersed in another liquid phase, producing double layered droplets.

The term **"elastic modulus (G')"** also known as the storage modulus refers to the ability of a viscoelastic material to store energy under shear. The elastic modulus is measured by oscillatory measurements using a rheometer with frequency scanning at low strain. Its value for the continuous phase is taken for the angular frequency corresponding to the maximum shear rate applied to the droplets during emulsification.

The term **"non-miscible"** refers to a liquid which does not combine or blend with another liquid, or which does not combine or blend immediately with another liquid.

The term **"non-miscible active agent"** refers to an active agent in liquid or solid form which is non-miscible with other ingredients in the dispersed phase. According to one embodiment, a non-miscible active agent is dispersed within the dispersed phase, either in liquid or solid form.

Non-limiting examples of non-miscible active agent include agents selected from a crosslinker, hardener, organometallic or inorganometallic catalyst, a colorant, a pigment, a perfume (such as the compounds published in the list of the International Fragrance Association IFRA), a flavouring, antioxidant, pH adjuster, preservative, a softener, a conditioner, an anti-discoloration agent (such as an ammonium derivative), an anti-foaming agent (such as an alcohol ethoxylate, an alkylbenzene sulfonate, a polyethylene ethoxylate, an alkylethoxysulfate or alkylsulfate), a brightening agent, also called a colour activator (such as a stilbene derivative, a coumarin derivative, a pyrazoline derivative, a benzoxazole derivative or a naphthalimide derivative), a biologically active compound such as an enzyme, a vitamin (eg vitamin A, B, C, D, E), a protein, a lipid, a probiotic, an alpha hydroxy acid, a ceramide, a polyphenol, hyaluronic acid, a plant extract, an emollient agent, a disinfectant agent, an antibacterial agent, an antiwear agent, a lubricating agent, an anti-UV agent or UV absorber, a pharmacologically active molecule, a medicament., a crop protection agent, a pheromone, a fertilizer, herbicide, insecticide, pesticide, fungicide, repellent or disinfectant for agrochemicals, a fire retardant, also called a flame retardant, (such as a brominated polyol like tetrabromobisphenol A, a halogenated or non-halogenated organophosphorus compound, a chlorinated compound, an aluminium trihydrate, an antimony oxide, a zinc borate, a red phosphorus, a melamine, or a magnesium dihydroxide), a photonic crystal, a photochromic complex, a phase change material that can absorb or release heat during phase transition and a mixture thereof.

The term **"monodisperse"** refers to a population of particles or droplets of varied size, wherein the standard deviation of the diameter distribution is lower than 25%.

The term **"Newtonian fluid"** refers to a fluid in which the viscous stresses arising from its flow, at every point, are linearly correlated to the local strain rate, i.e. the rate of change of its deformation over time. In other words, the viscosity of a Newtonian fluid is constant and independent of the shear rate applied and the elastic modulus can be neglected compared to the storage modulus. Some fluids may exhibit a complex behavior under shear, with ranges of shear rates where the viscosity is constant and ranges of shear rates where the viscosity is shear rate dependent. According to one embodiment, a fluid is considered a Newtonian fluid when its viscosity is constant at all shear rates applied during the mixing step of the emulsion. According to one embodiment, a fluid is considered a Newtonian fluid when its viscosity is constant for shear rates ranging from $0.1$ s$^{-1}$ to $1\,000$ s$^{-1}$. According to one embodiment, a fluid is considered a Newtonian fluid when its viscosity is constant for shear rates ranging from $0.1$ s$^{-1}$ to $500$ s$^{-1}$. According to one embodiment, a fluid is considered a Newtonian fluid when the relative variation of its viscosity is inferior to 10% for shear rates ranging from 0.1 s$^{-1}$ to 200 s$^{-1}$.

The term **"initiator"** refers to a compound capable of initiating or accelerating a polymerization reaction.

The term **"polydisperse"** refers to a population of particles or droplets of varied size, wherein the standard deviation of the diameter distribution is superior to 25%.

The term **"ranging from x to y"** refers to a range that includes both x and y values as well as every value between x and y.

The term **"size-controlled"** refers to a population of particles or droplets whose average size can be predicted from the physical parameters of the emulsion using equation (1) below.

The term **"viscous modulus (G")"** also known as the loss modulus refers to the ability of the viscoelastic material to dissipate energy under shear. The viscous modulus is measured by oscillatory measurements using a rheometer with frequency scanning at low strain. Its value for the continuous phase is taken for the angular frequency corresponding to the maximum shear rate applied to the droplets during emulsification.

The term **"viscoelasticity"** refers to the property of a material which, under the effect of shear and deformation, has both the characteristics of a purely elastic material, i.e. it is capable of storing energy, and the characteristics of a purely viscous material, i.e. it is also capable of dissipating energy.

The term **"viscoelastic continuous phase"** refers to a continuous phase in an emulsion that exhibits viscoelasticity.

## DETAILED DESCRIPTION

*Emulsion*

This invention relates to an emulsion comprising size-controlled droplets dispersed in a viscoelastic continuous phase, said viscoelastic continuous phase comprising at least one polymer in solution in a solvent.

According to one embodiment, the emulsion is a single emulsion or a multiple emulsion. According to one embodiment, the emulsion is a double emulsion.

*Dispersed phase*

According to one embodiment, the dispersed phase comprises at least one crosslinkable material and optionally at least one initiator.

According to one embodiment, the at least one crosslinkable material is selected from at least one monomer or polymer, at least one cross-linking agent and a mixture thereof.

According to one embodiment, the monomer or polymer is selected from monomers and polymers comprising at least one reactive function selected from the group consisting of acrylate, methacrylate, vinyl ether, N-vinyl ether, mercaptoester, thiolene, siloxane, epoxy, oxetane, urethane, isocyanate and peroxide functions. According to one embodiment, the monomer or polymer is selected from monomers and polymers carrying at least one of the above-mentioned reactive functions and further carrying at least one function selected from the group consisting of primary, secondary and tertiary alkylamine functions, quaternary amino functions, sulphate, sulphonate, phosphate, phosphonate, carboxylate, hydroxyl, halogen and a mixture thereof.

According to one embodiment, the monomer or polymer is selected from polyethers, polyesters, polyurethanes, polyanhydride, polycarbonate, polyureas, polyethylene glycols, polypropylene glycols, polyamides, polyacetals, polyimides, polyolefins, polysulfides, polydimethylsiloxanes, polysaccharides and a mixture thereof. According to one embodiment, said monomer or polymer further carries at least one reactive function selected from the group consisting of acrylate, methacrylate, vinyl ether, N-vinyl ether, mercaptoester, thiolene, siloxane, epoxy, oxetane, urethane, isocyanate and peroxide functions.

Non-limiting examples of monomers or polymers include compounds selected from poly(2-(1-naphthyloxy)-ethyl acrylate), poly(2-(2-naphthyloxy)-ethyl acrylate), poly(2-(2-naphthyloxy)-ethyl methacrylate), polysorbitol dimethacrylate, polyacrylamide, poly((2-(1-naphthyloxy) ethanol), poly(2-(2-naphthyloxy) ethanol), poly(1-chloro-2,3-epoxypropane), poly(n-butyl isocyanate), poly(N-vinyl carbazole), poly(N-vinyl pyrrolidone), poly(p-benzamide), poly(p-chlorostyrene), poly(p-methyl styrene), poly(p-phenylene oxide), poly(p-phenylene sulfide), poly(N-(methacryloxyethyl)succinimide), polybenzimidazol, polybutadiene, polybutylene terephthalate, polychloro trifluoro ethylene, polyether imide, polyether ketone, polyether sulfone, polyhydridosilsesquioxane, poly(m-phenylene isophthalamide), poly(methyl 2-acrylamido-2-methoxyacetate), poly(2-acrylamido-2-methylpropanesulfonic acid), poly-mono-butyl maleate, polybutylmethacrylate, poly(N-tert-butylmethacrylamide), poly(N-n-butylmethacrylamide), polycyclohexylmethacrylamide, poly(m-xylenebisacrylamide 2,3-dimethyl-1,3-butadiene,N, N-dimethylmethacrylamide), poly(n-butyl methacrylate), poly(n-butyl methacrylate), poly(cyclohexyl methacrylate), polyisobutyl methacrylate, poly(4-cyclohexylstyrene), polycyclol acrylate, polycyclol methacrylate, polydiethyl ethoxymethylene malonate, poly(2,2,2-trifluoroethyl methacrylate), poly(1,1,1 - trimethylolpropane trimethacrylate), polymethacrylate, poly(N,N-dimethylaniline, dihydrazide), poly(isophthalic dihydrazine), polydimethyl benzilketal, epichlorohydrin, poly(ethyl-3-yl),3-diethoxyacrylate), poly(ethyl-3,3-dimethylacrylate), poly(ethyl vinyl ketone), poly(vinyl ethyl ketone), poly(penten-3- one), polyformaldehyde poly(diallyl acetal), polyfumaronitrile, polyglyceryl propoxy triacrylate, polyglyceryl trimethacrylate, polyglycidoxypropyltrimethoxysilane, polyglycidyl acrylate, poly(n-heptyl acrylate), poly(n-heptyl acrylic acid ester), poly(n-heptyl methacrylate), poly(3-hydroxypropionitrile), poly(2-hydroxypropyl acrylate), poly(2-hydroxypropyl methacrylate), poly(N-(methacryloxyethyl)phthalimide), poly(1,9-nonanediol diacrylate), poly(1,9- nonanediol dimethacrylate), poly(N-(n-propyl) acrylamide), poly(ortho-phthalic acid), poly(iso-phthalic acid), poly(1 ,4-benzenedicarboxylic acid), poly(1,3-benzenedicarboxylic acid), poly(phthalic acid), poly(mono-2- acryloxyethyl ester), poly-terephthalic acid, polyphthalic anhydride, polyethylene glycol diacrylate, polyethylene glycol methacrylate, polyethylene glycol dimethacrylate, poly(isopropyl acrylate), polysorbitol pentaacrylate, polyvinyl bromoacetate, polychloroprene, poly(di-n-hexyl silylene), poly(di-n-propyl siloxane), polydimethyl silylene, polydiphenyl siloxane, polyvinyl propionate, polyvinyl triacetoxysilane, polyvinyl tris-tert-butoxysilane, polyvinyl butyral, polyvinyl alcohol, polyvinyl acetate, polyethylene co-vinyl acetate, poly(bisphenol-A polysulfone), poly(1,3-dioxepane), poly(1,4-phenylene vinylene), poly(2,6-dimethyl-1-A-phenylene oxide), poly(4-hydroxybenzoic acid), poly(4-methyl-1-pentene), poly(4-vinyl pyridine), polymethylacrylonitrile, polymethylphenylsiloxane, polymethylsilmethylene, polymethylsilsesquioxane, poly(phenylsilsesquioxane), poly(pyromellitimide-1,4-diphenyl ether), polytetrahydrofuran, polythiophene, poly(trimethylene oxide), polyacrylonitrile, polyether sulfone, polyethylene-co-vinyl acetate, poly(perfluoroethylene propylene), poly(perfluoralkoxyl alkane), poly(styrene-acrylonitrile) and a mixture thereof.

According to one embodiment, the cross-linking agent is selected from molecules carrying at least two reactive functions selected from acrylate, methacrylate, vinyl ether, N-vinyl ether, mercaptoester, thiolene, siloxane, epoxy, oxetane, urethane, isocyanate and peroxide functions.

Non-limiting examples of cross-linking agent include compounds selected from 1,6-hexanediol diacrylate, 1,6-hexanediol dimethacrylate, polyethylene glycol dimethacrylate, 1,9-nonanediol dimethacrylate, 1 ,4-butanediol dimethacrylate, 2,2-bis(4-methacryloxyphenyl) propane, 1 ,3-butanediol dimethacrylate, 1,10-decanediol dimethacrylate, bis(2-methacryloxyethyl)N,N'-1,9-nonylene biscarbamate, 1,4-butanediol diacrylate, ethylene glycol diacrylate, 1,5-pentanediol dimethacrylate, 1 ,4-phenylene diacrylate, allyl methacrylate, N,N'-methylenebisacrylamide, 2,2- bis[4-(2-hydroxy-3-methacryloxypropoxy)phenyl]propane, tetraethylene glycol diacrylate, ethylene glycol dimethacrylate, diethylene glycol diacrylate, triethylene glycol diacrylate, triethylene glycol dimethacrylate, polyethylene glycol diglycidyl ether, N, N-diallylacrylamide, 2,2-bis[4-(2-acryloxyethoxy) phenyl]propane, glycidyl methacrylate, dipentaerythritol pentaacrylate, 1,1,1 - trimethylolpropane triacrylate, 1,1,1-trimethylolpropane trimethacrylate, ethylenediamine tetramethacrylate, pentaerythritol triacrylate, pentaerythritol tetraacrylate, propargyl methacrylate, 2-cyanoethyl acrylate, tricyclodecane dimethanol diacrylate, hydroxypropyl methacrylate, N-acryloxysuccinimide, N-(2-hydroxypropyl)methacrylamide, N-(3-aminopropyl)methacrylamide hydrochloride, N-(t-BOC-aminopropyl)methacrylamide, 2-aminoethyl methacrylate hydrochloride, monoacryloxyethyl phosphate, o-nitrobenzyl methacrylate, acrylic anhydride, 2-(tert-butylamino)ethyl methacrylate, N,N- diallylacrylamide, glycidyl methacrylate, 2-hydroxyethyl acrylate, 4-(2-acryloxyacehoxy)-2-hydroxybenzophenone, N-(phthalimidomethyl)acrylamide, cinnamyl methacrylate and a mixture thereof.

According to one embodiment, the at least one initiator is a photo-initiator capable of initiating or accelerating a polymerization under the effect of light radiation, selected from $\alpha$-hydroxyketones, $\alpha$-aminoketones, aromatic ketones, thioxanthones, quinones, $\alpha$-dicarbonyl derivatives, acylphosphine oxides, and a mixture thereof.

Non-limiting examples of photo-initiators include compounds selected from 2-hydroxy-2-methyl-1-phenyl-1-propanone, marketed for example under the names DAROCUR® 1 173 and 4265, IRGACURE® 184, 2959, and 500 by BASF, and ADDITOL® CPK by CYTEC; 2-benzyl-2-dimethylamino-1 -(4-morpholinophenyl)-butanone-1, marketed for example under the names IRGACURE® 907 and 369 by BASF; aromatic ketones marketed for example under the name ESACURE® TZT by LAMBERTI; thioxanthones marketed for example under the name ESACURE® ITX by LAMBERTI; benzyldimethylketal marketed as IRGACURE® 651 by BASF; bis-acylphosphine oxides (BAPO) marketed for example under the names IRGACURE® 819, 1700, and 1800, DAROCUR® 4265, LUCIRIN® TPO, and LUCIRIN® TPO-L by the company BASF; benzophenone, phenyl glyoxylates, such as phenyl glyoxylic acid methyl ester, oxime esters, such as [1-(4-phenylsulfanylbenzoyl)heptylideneamino]benzoate, sulfonium salts, iodonium salts, and oxime sulfonates.

According to one embodiment, the at least one initiator is a thermal initiator capable of initiating or accelerating a polymerization reaction under the effect of heat. Non-limiting examples of thermal initiators include azo compounds such as 2,2'-azobis(isobutyronitrile), 2,2'-azobis-2-methylbutyronitrile or 2,2'-azobis-2,4-dimethylvaleronitrile; organic peroxides such as benzoyl peroxide or dicumyl peroxide; dicyandiamide, cyclohexyl tosylate,

According to one embodiment, the at least one initiator is a redox initiator capable of initiating or accelerating a polymerization reaction by decomposition into radicals. Non-limiting examples of redox initiators include metal ion based reducing agents coupled to a peroxide, a persulfate or a disulfide and aromatic tertiary amine reducing agents coupled to a peroxide.

According to one embodiment, the dispersed phase comprises from 15% to 95% by weight of at least one crosslinkable material in respect to the total weight of the dispersed phase. According to one embodiment, the dispersed phase comprises from 5% to 30% by weight of at least one crosslinking agent in respect to the total weight of the dispersed phase. According to one embodiment, the dispersed phase comprises from 0.1% to 5% by weight of at least one initiator in respect to the total weight of the dispersed phase.

According to one embodiment, the dispersed phase further comprises from at least one non-miscible active agent. According to one embodiment, the dispersed phase further comprises from 5% to 80% by weight of at least one non-miscible active agent in respect to the total weight of the dispersed phase.

According to one embodiment, the at least one non-miscible active agent is selected from a crosslinker, hardener, organometallic or inorganometallic catalyst, a colorant, a pigment, a perfume (such as the compounds published in the list of the International Fragrance Association IFRA), a flavouring, antioxidant, pH adjuster, preservative, a softener, a conditioner, an anti-discoloration agent (such as an ammonium derivative), an anti-foaming agent (such as an alcohol ethoxylate, an alkylbenzene sulfonate, a polyethylene ethoxylate, an alkylethoxysulfate or alkylsulfate), a brightening agent, also called a colour activator (such as a stilbene derivative, a coumarin derivative, a pyrazoline derivative, a benzoxazole derivative or a naphthalimide derivative), a biologically active compound such as an enzyme, a vitamin (eg vitamin A, B, C, D, E), a protein, a lipid, a probiotic, an alpha hydroxy acid, a ceramide, a polyphenol, hyaluronic acid, a plant extract, an emollient agent, a disinfectant agent, an antibacterial agent, an antiwear agent, a lubricating agent, an anti-UV agent or UV absorber, a pharmacologically active molecule, a medicament., a crop protection agent, a pheromone, a fertilizer, herbicide, insecticide, pesticide, fungicide, repellent or disinfectant for agrochemicals, a fire retardant, also called a flame retardant, (such as a brominated polyol like tetrabromobisphenol A, a halogenated or non-halogenated organophosphorus compound, a chlorinated compound, an aluminium trihydrate, an antimony oxide, a zinc borate, a red phosphorus, a melamine, or a magnesium dihydroxide), a photonic crystal, a photochromic complex, a phase change material that can absorb or release heat during phase transition and a mixture thereof.

According to one embodiment, the proportion of dispersed phase is between 5% and 50%, by weight in respect to the total weight of the emulsion.

According to one embodiment, the viscosity ($\eta_i$) of the dispersed phase at 25°C is between 20 mPa.s and 50 000 mPa.s. According to one embodiment, the viscosity of the dispersed phase is between 20 mPa.s and 40 000 mPa.s. According to one embodiment, the viscosity of the dispersed phase is between 100 mPa.s and 40 000 mPa.s. According to one embodiment, the viscosity of the dispersed phase is between 20 mPa.s and 3 000 mPa.s. According to one embodiment, the viscosity of the dispersed phase is between 3 000 mPa.s and 40 000 mPa.s. According to one embodiment, the viscosity of the dispersed phase is between 10 000 mPa.s and 40 000 mPa.s.

The viscosity of the dispersed phase may be measured by methods well-known to a skilled person in the art, e.g. by means of a Anton Paar rheometer MCR 92 equipped with a 25 mm diameter parallel plate geometry or a 50 mm cone plate geometry with a 2-degree angle, and a temperature control cell set at 25°C. The viscosity value is read at a shear rate of 0.1 s$^{-1}$.

## *Continuous phase*

According to one embodiment, the continuous phase is a viscoelastic continuous phase. According to one embodiment, the viscoelastic continuous phase comprises at least one polymer in solution in at least one solvent. According to one embodiment, the viscoelastic continuous phase comprises at least one polymer in solution in at least one solvent at a concentration ranging from 0.5 to 80 % by weight in respect to the total weight of the continuous phase. In one embodiment, the viscoelastic continuous phase comprises at least one polymer in solution in at least one solvent at a concentration ranging from 0.5 to 40 % (w/w). In one specific embodiment, the viscoelastic continuous phase comprises at least one polymer in solution in at least one solvent at a concentration ranging from 1 to 20 % (w/w).

According to one embodiment, the solution of at least one polymer represents at least 50% of the viscoelastic continuous phase. According to one embodiment, the solution of at least one polymer represents at least 60% of the viscoelastic continuous phase. According to one embodiment, the solution of at least one polymer represent at least 70% of the viscoelastic continuous phase. According to one embodiment, the solution of at least one polymer represents at least 80% of the viscoelastic continuous phase. According to one embodiment, the solution of at least one polymer represents at least 90% of the viscoelastic continuous phase. According to one embodiment, the solution of at least one polymer represents at least 95% of the viscoelastic continuous phase. According to a preferred embodiment, the solution of at least one polymer represents 100% of the viscoelastic continuous phase.

According to one embodiment, the viscoelastic continuous phase comprises at least one polymer with a molecular weight greater than 5 000 g.mol$^{-1}$. According to one embodiment, the viscoelastic continuous phase comprises at least one polymer with a molecular weight between 10 000 g.mol$^{-1}$ and 500 000 g.mol$^{-1}$; preferably, between 20 000 g.mol$^{-1}$ and 300 000 g.mol$^{-1}$; most preferably between 50 000 g.mol$^{-1}$ and 200 000 g.mol$^{-1}$.

According to one embodiment, the at least one polymer is selected from the following polymers and a mixture thereof:

- cellulose derivatives, such as cellulose ethers, e.g. methyl cellulose, ethyl cellulose, hydroxyethyl cellulose, methylhydroxyethyl cellulose, ethylhydroxyethyl cellulose, carboxymethyl cellulose, hydroxypropyl cellulose or methylhydroxypropyl cellulose;
- polyacrylates (also called carbomers), such as polyacrylic acid (PAA), polymethacrylic acid (PMAA), poly(hydroxyethyl methacrylate) (pHEMA) or poly(N-2-hydroxypropyl methacrylate) (pHPMA);
- polyacrylamides such as poly(N-isopropylacrylamide) (NIPAM); polyvinylpyrrolidone (PVP) and its derivatives or polyvinyl alcohol (PVA) and its derivatives;
- poly(ethylene glycol), poly(propylene glycol) and their derivatives, such as poly(ethylene glycol) acrylate/methacrylate, poly(ethylene glycol) diacrylate/dimethacrylate or polypropylene carbonate;
- polysaccharides such as carrageenan, locust bean gum or tara gum, dextran, xanthan gum, chitosan, agarose, hyaluronic acids, gellan gum, guar gum, gum arabic, tragacanth gum, diutane gum, oat gum, karaya gum, ghatti gum, curdlan gum, pectin, konjac gum or starch;
- protein derivatives such as gelatin, collagen, fibrin, polylysine, albumin or casein;
- silicone derivatives such as polydimethylsiloxane (also called dimethicone), alkyl silicones, aryl silicones, alkyl aryl silicones, polyethylene glycol dimethicones or polypropylene glycol dimethicone;
- waxes such as diester waxes (alkanediol diesters, hydroxylacid diesters), triester waxes (triacylglycerols, alkane-1,2-diol triesters, $\omega$-hydroxy acid and fatty acid triesters), esters of hydroxymalonic acid, fatty acid and alcohol, triesters of hydroxylacids, fatty acid and fatty alcohol, triesters of fatty acid, hydroxylacid and diol or polyester waxes (fatty acid polyesters);
- fatty acid esters (which can for example be used as waxes) such as cetyl palmitate, cetyl octanoate, cetyl laurate, cetyl lactate, cetyl isononanoate, cetyl stearate, stearyl stearate, myristyl stearate, cetyl myristate, isoscetyl stearate, glyceryl trimyristate, glyceryl tripalmitate, glyceryl monostearate or glyceryl cetyl palmitate;
- fatty acids (which can for example be used as waxes) such as cerotic acid, palmitic acid, stearic acid, dihydroxystearic acid, behenic acid, lignoceric acid, arachidic acid, myristic acid, lauric acid, tridecyclic acid, pentadecyclic acid, margaric acid, non-adecyclic acid, phenicosylic acid, tricosylic acid, pentacosylic acid, heptacosylic acid, montanic acid or nonacosylic acid;
- fatty acid salts including aluminium salts of fatty acids such as aluminium stearate or hydroxyl aluminium bis(2-ethylhexanoate);
- polyurethanes and their derivatives;
- styrenic polymers such as styrene butadiene; and
- polyolefins such as polyisobutene.

According to a preferred embodiment, the at least one polymer is selected from cellulose derivatives, polyacrylates, polysaccharides, silicone derivatives and a mixture thereof.

According to a more preferred embodiment, the at least one polymer is selected from chitosan, carboxymethyl cellulose, methyl cellulose, guar, konjac, polyacrylic acid, polyethylene oxide and a mixture thereof.

According to one embodiment, the at least one polymer is not an alginate. Alginates are salts of alginic acid (algin), a natural linear polymer based on two monomeric units, $\beta$-D-mannuronic acid (M) and $\alpha$-L-guluronic acid (G), covalently linked together by $\beta$(1-4) glycosidic bonds. The molar ratio M/G can vary widely as a function of the algal source, for example from 0.2 to 4. Most alginates are extracted from brown seaweeds, in particular from Laminaria, Macrocystis or Ascophyllum. The alginate salts of interest are sodium alginate, calcium alginate, ammonium alginate and potassium alginate.

According to one embodiment, said viscoelastic continuous phase does not comprise a surfactant.

Surfactants are molecules well known by the skilled artisan to stabilize an emulsion by slowing down or stopping the mechanisms of emulsion ageing such as coalescence and growth. Surfactants are selected from anionic surfactants such as alkali metal alkylbenzenesulphonates, alkali metal alkylsulphates such as sodium dodecyl sulphate, alkali metal alkyl ether sulphates, alkali metal alkylaryl ether sulphates and alkali metal dioctyl sulphosuccinates; cationic surfactants such as dialkyl ($C_{10}$-$C_{30}$) benzyldimethylammonium halides and polyethoxylated quaternary ammonium salts; amphoteric surfactant such aN-alkyl ($C_{10}$-$C_{22}$) betaines, N-alkyl ($C_{10}$-$C_{22}$) amidobetaines, ($C_{10}$-$C_{22}$)-alkyl imidazolines and asparagine derivatives; amphoteric surfactants such as alkylamphoacetates; or non-ionic surfactants such as oxyethylenated and oxypropylenated block polymers (poloxamers), polyethoxylated fatty acids, sorbitan esters, polyethoxylated sorbitan esters, polyethoxylated alkylphenols, polyethoxylated fatty alcohols, derivatives of polyethylene glycol and of a mixture of glycerides (mono-, di- and tri-glycerides) of fatty acids; polyethylene glycol derivatives of a mixture of glycerides (alkoxylated triglycerides) of vegetable origin, alkylpolyglycosides, polyethoxylated or polyglycerol fatty amides, polyglycerol alcohols and alphadiols.

According to one embodiment, said viscoelastic continuous phase comprises exactly one polymer in solution in at least one solvent.

According to one embodiment, the at one solvent is selected from water; alcohols such as methanol, ethanol, 1-propanol, 2-propanol, 1-butanol, t-butanol, hexanol, octanol, phenol, glycerol; ketones such as acetone, methylethyl ketone; low molecular weight carboxylic acids such as acetic acid; low molecular weight esters such as ethyl acetate; glycols such as ethylene glycol; propylene carbonate; acetonitrile; dimethylsulfoxide; tetrahydrofurane.

According to one embodiment, the viscosity ($\eta$) of the viscoelastic continuous phase at 25°C is higher than the viscosity ($\eta_i$) of the dispersed phase at 25°C. According to the one embodiment, the viscosity of the viscoelastic continuous phase at 25°C is between 2 000 mPa.s and 10 000 000 mPa.s. According to one embodiment, the viscosity of the viscoelastic continuous phase is between 5 000 mPa.s and 1 000 000 mPa.s. According to one embodiment, the viscosity of the viscoelastic continuous phase is between 10 000 mPa.s and 500 000 mPa.s.

Advantageously, the high viscosity of the viscoelastic continuous phase allows a kinetic stability of the emulsion.

The viscosity of the viscoelastic continuous phase may be measured by methods well known to the skilled person in the art, e.g. by means of a Anton Paar rheometer MCR 92 equipped with a 25 mm diameter parallel plate geometry or a 50 mm cone plate geometry with a 2-degree angle, and a temperature control cell set at 25°C. The viscosity value is read at a shear rate of 0.1 s$^{-1}$.

According to one embodiment, the viscoelastic continuous phase is characterized by the ratio between the viscous modulus (G") and the elastic modulus (G'), whose variation will impact the behavior of the viscoelastic continuous phase and will allow to define the domains in which no droplets, monodisperse or polydisperse size-controlled droplets are formed.

According to one embodiment, the viscoelastic continuous phase presents a ratio between the viscous modulus (G") and the elastic modulus (G') ranging from 0.2 to 10, and the formed droplets are size-controlled and polydisperse or size-controlled and monodisperse. According to one embodiment, the viscoelastic continuous phase presents a ratio between the viscous modulus (G") and the elastic modulus (G') ranging from 0.2 to 5, and the formed droplets are size-controlled and polydisperse or size-controlled and monodisperse.

According to one embodiment, the ratio between the viscous modulus (G") and the elastic modulus (G') ranges from 0.2 to 1, and polydisperse size-controlled droplets are formed.

According to a preferred embodiment, the ratio between the viscous modulus (G") and the elastic modulus (G') ranges from 1 to 5, and monodisperse size-controlled droplets are formed. According to a more preferred embodiment, the ratio between the viscous modulus (G") and the elastic modulus (G') ranges from 1 to 3, and monodisperse size-controlled droplets are formed.

Advantageously, the variation of the diameter (D) of the formed droplets is function of the G'/G" ratio at the angular frequency corresponding to the shear applied during emulsification. According to one embodiment, the mean diameter D of said droplets varies linearly as function of equation (1) in the domain 0.2 < G"/G' < 10.

$$(1)\ D \sim \left(G'/G''\right)^{1.5}$$

According to one embodiment, the droplets have a mean diameter inferior to 100 $\mu$m. According to one embodiment, the droplets have a mean diameter ranging from 1 to 70 $\mu$m. According to one embodiment, the droplets have a mean diameter ranging from 20 to 70 $\mu$m. According to a preferred embodiment, the droplets have a mean diameter ranging from 1 to 20 $\mu$m.

According to another embodiment, when said viscoelastic continuous phase comprises exactly one polymer in solution in at least one solvent, the degree of entanglements of the polymer chains in solution can be characterized and said degree of entanglements is correlated with the G"/G' ratio. A higher degree of entanglement leads to an increase of the elastic modulus (G') of the viscoelastic continuous phase. The degree of entanglements depends on the molecular weight of the polymer and its concentration (C) in the solvent. The intrinsic viscosity, depending only on the molecular weight of the polymer, can be approximated to the viscosity of the polymer solution at 1% concentration ($\eta_{(1\%)}$) by weight in respect to the total weight of the viscoelastic continuous phase. $\eta_{(1\%)}$ is intrinsic to the chosen polymer for a given solvent.

According to one embodiment, the product $\eta_{(1\%)}$.C is used to characterize the degree of entanglements of the polymer solution. According to one embodiment, the G"/G' ratio depends on the degree of entanglements of the polymer chains in the solution, whose variation will allow to define the domains in which no droplets, monodisperse or polydisperse size-controlled droplets are formed.

Advantageously, the G"/G' ratio varies linearly as function of equation (2):

$$(2)\ G''/G' \sim \left(\eta_{(1\%)}.C\right)^{-0.3}$$

According to one embodiment, the product $\eta_{(1\%)}$.C ranges from 100 mPa.s to 100 000 mPa.s, in this range polydisperse and monodisperse size-controlled droplets are formed.

According to one embodiment, the product $\eta_{(1\%)}$.C ranges from 1 000 mPa.s to 100 000 mPa.s, in this range polydisperse size-controlled droplets are formed.

According to a preferred embodiment, the product $\eta_{(1\%)}$.C ranges from 100 mPa.s to 1 000 mPa.s, in this range monodisperse size-controlled droplets are formed.

According to one embodiment, the droplets are size-controlled polydisperse droplets. According to a preferred embodiment, the droplets are size-controlled monodisperse droplets.

According to one embodiment, a population of monodisperse droplets has a standard deviation of the diameter distribution less than 80%. According to one embodiment, a population of monodisperse droplets has a standard deviation of the diameter distribution less than 50%. According to a preferred embodiment, a population of monodisperse droplets has a standard deviation of the diameter distribution less than 25%. The standard deviation of the diameter distribution may be measured by methods well known to the skilled person in the art, e.g. by a light scattering technique such as a Mastersizer 3000 equipped with a hydro SV measuring cell, or by image analysis of optical microscopy pictures, or by image analysis of electronic microscopy pictures.

According to one embodiment, the viscoelastic continuous phase further comprises at least one Newtonian fluid. The at least one Newtonian fluid is either a pure ingredient or an ingredient dissolved in a solvent that is miscible with the continuous phase solvents.

Advantageously, the at least one Newtonian fluid allows a skilled artisan to control the G"/G' ratio and thus the mean diameter D of the droplets.

According to one embodiment, the viscoelastic continuous phase comprises at least one Newtonian fluid in solution at a proportion ranging from 0.05 to 50% by weight in respect to the total weight of the continuous phase. According to one embodiment, the viscoelastic continuous phase comprises at least one Newtonian fluid in solution at a proportion ranging from 0.1 to 20%. According to one embodiment, the viscoelastic continuous phase comprises at least one Newtonian fluid in solution at a proportion ranging from 0.1 to 5%. According to one embodiment, the viscoelastic continuous phase comprises at least one Newtonian fluid in solution at a proportion ranging from 20 to 50%.

According to one embodiment, the G"/G' ratio varies linearly as function of the concentration of the at least one Newtonian fluid comprised into the viscoelastic continuous phase.

According to one embodiment, the at least one Newtonian fluid is selected from vegetable oils such as avocado oil, canola oil, grape seed oil, nuts oil, olive oil, rapeseed oil, rice bran oil, safflower oil, sesame oil, soybean oil, sunflower oil; glycerol and glycerol derivatives; mineral oils; polyurethanes, polyacrylates, polyethers and cellulose ethers have a linear chain and a molecular weight ranging from 5 000 to 100 000 g/mol, preferably from 10 000 to 50 000 g/mol and most preferably from 15 000 to 30 000 g/mol, such as ethoxylate urethane polymers, hydroxyethyl cellulose; and a mixture thereof.

*Process*

The invention also relates to a process for the fabrication of an emulsion as described herein above, comprising the step of mixing the emulsion, thus submitting to shear a population of droplets dispersed in the viscoelastic continuous phase, said viscoelastic continuous phase comprising at least one polymer in solution in at least one solvent.

The embodiments defining the above emulsion apply *mutatis mutandis* to the above process.

According to one embodiment, the viscosity of the continuous phase ($\eta$) is higher than the viscosity of the dispersed phase ($\eta_i$), preferably, the $\eta_i/\eta$ ratio belongs to the range $0.01< \eta_i/\eta <1$ in order to homogeneously rupture mother droplets into smaller daughter droplets under shear.

As described herein above, it was found by the applicant that the variation of the G"/G' ratio will impact the behavior of the viscoelastic continuous phase and will allow to define the domain in which no droplets, monodisperse or polydisperse size-controlled droplets are formed.

According to one embodiment, the ratio between the viscous modulus (G") and the elastic modulus (G') belongs to the domains as described above for the viscoelastic continuous phase, in which polydisperse and/or monodisperse droplets are formed and the mean diameter D of said droplets varies linearly as function of equation (1) mentioned above in the domain ranging from 0.2 to 10.

According to one embodiment, the formed droplets have a mean diameter inferior to 100 $\mu$m. According to one embodiment, the formed droplets have a mean diameter ranging from 1 to 70 $\mu$m. According to one embodiment, the formed droplets have a mean diameter ranging from 20 to 70 $\mu$m. According to a preferred embodiment, the formed droplets have a mean diameter ranging from 1 to 20 $\mu$m.

As described above, the G"/G' ratio is correlated with the degree of entanglements ($\eta_{(1\%)}$.C) of the polymer chains in solution. It was found that the G"/G' ratio varies linearly as function of equation (2) mentioned above and that its variation allows to define the domain in which no droplets, monodisperse or polydisperse size-controlled droplets are formed, based on the ability to fragmentize the mother droplets.

According to one embodiment, the product $\eta_{(1\%)}$.C belongs to the domains as described above, in which polydisperse and/or monodisperse size-controlled droplets are formed.

According to one embodiment, shear is applied to the emulsion in order to fragmentize the dispersed droplets. According to one embodiment, a shear rate ranging from 1 to 20 000 $s^{-1}$ is applied. According to a preferred embodiment, a shear rate ranging from 1 to 1 000 $s^{-1}$ is applied.

According to one embodiment, formed droplets are size-controlled, polydisperse droplets. According to a preferred embodiment, formed droplets are size-controlled, monodisperse droplets.

According to one embodiment, the formed population of monodisperse droplets has a standard deviation of the diameter distribution less than 80%. According to one embodiment, the formed population of monodisperse droplets has a standard deviation of the diameter distribution less than 50%. According to a preferred embodiment, the formed population of monodisperse droplets has a standard deviation of the diameter distribution less than 25%.

According to one embodiment, the process further comprises the addition of at least one Newtonian fluid in solution into the viscoelastic continuous phase at a concentration ranging from 0.05 to 50 % by weight in respect to the total weight of the continuous phase. Advantageously, the addition of at least one Newtonian fluid allows to control the G"/G' ratio and thus the mean diameter D of the formed droplets as the G"/G' ratio varies linearly as function of the concentration of the Newtonian fluid comprised into the viscoelastic continuous phase.

According to one embodiment, the mean diameter of the formed droplets is measured just after the shear is applied. The mean diameter may be measured by methods well known to the skilled person in the art, e.g. by a light scattering technique such as a Mastersizer 3000 equipped with a hydro SV measuring cell, or by image analysis of optical microscopy pictures, or by image analysis of electronic microscopy pictures.

Due to the transient nature of the emulsion (especially if the continuous phase contains no surfactant), ageing mechanisms start to broaden the size distribution of the formed droplets after the mixing is stopped. Such ageing mechanisms are well-known to the skilled artisan and include for example coalescence and growth.

According to one embodiment, the process further comprises a step of cross-linking the formed droplets as soon as mixing is stopped. Advantageously, it allows to freeze the system in order to measure the exact mean diameter of the formed droplets that is resulting from the application of a given shear. The mean diameter may be measured by methods well known to the skilled person in the art, e.g. by a light scattering technique such as a Mastersizer 3000 equipped with a hydro SV measuring cell, or by image analysis of optical microscopy pictures, or by image analysis of electronic microscopy pictures.

According to one embodiment, cross-linking is performed by subjecting the emulsion to a polymerization step. According to one embodiment, cross-linking is performed by subjecting the emulsion to a photopolymerization consisting in exposing the emulsion to a light source capable of initiating the polymerization of the formed droplets. According to one embodiment, the light source is a source of UV light with a wavelength ranging from 100 nm to 400 nm. According to one embodiment, cross-linking is performed by subjecting the emulsion to a thermal polymerization consisting in exposing the emulsion to heat.

*Use of the emulsion*

The invention also relates to the use of the emulsion as described hereinabove in applications requiring size-controlled droplets, preferably size-controlled monodisperse droplets.

According to one embodiment, the emulsion as described hereinabove is used in formulations requiring size-controlled droplets, preferably size-controlled monodisperse droplets. According to one embodiment, the emulsion as described herein above is used in formulations requiring limited or no use of surfactants.

According to one embodiment, the emulsion of the present invention is used in the cosmetic and personal care field, for example, in formulation of washing hygiene products, such as shampoos, shower gels, toothpastes, shaving creams or formulations of beauty products such as face and body creams, solar creams or make-up.

According to one embodiment, the emulsion of the present invention is used in the home and fabric care field, for example, in detergent compositions for household or professional use, such as detergents, softeners, dishwashing products or cleaning products.

According to one embodiment, the emulsion of the present invention is used in the pharmaceutical field.

According to one embodiment, the emulsion of the present invention is used in agriculture and crop protection, for example, in phytosanitary compositions, such as antiseptics, herbicides, insecticides or fertilizers.

According to one embodiment, the emulsion of the present invention is used in the field of paints, coatings, adhesives and sealants.

According to one embodiment, the emulsion of the present invention is used in the field of lubricants or fuels.

According to one embodiment, the emulsion of the present invention is used in the field of materials, plastics, composites and foams.

According to another embodiment, if a step of cross-linking of the formed droplets is performed, the formed size-controlled microparticles may be used for the encapsulation of various non-miscible active agents as per the definition above.

## BRIEF DESCRIPTION OF THE DRAWINGS

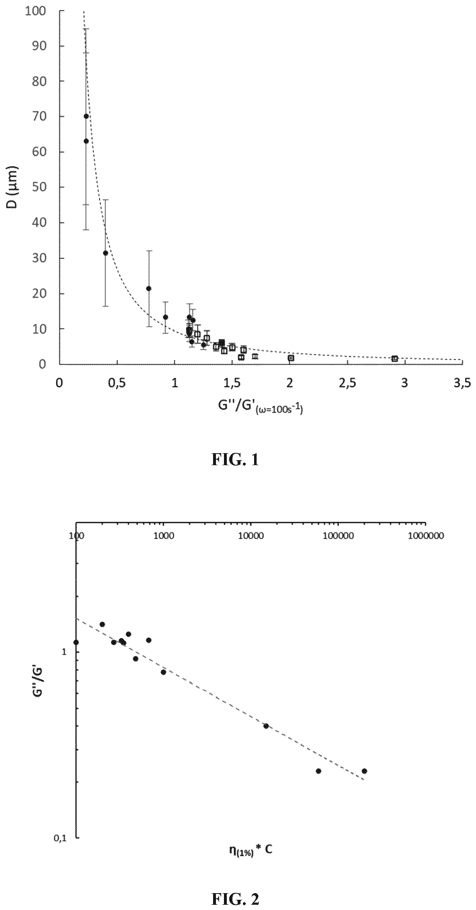

**Figure 1** is a graph showing the variation of the mean diameter (D) and size distribution of the formed droplets as a function of G"/G' ratio at the working angular frequency for materials of Table 1 (filled circles) and Table 2 (open squares). Data points correspond to the mean diameter (D) and error bars correspond to the standard deviation of the size distribution. The dotted line corresponds to the equation (1).

**Figure 2** is a graph showing the evolution of the G"/G' ratio as a function of the product $\eta_{(1\%)}$.C for the materials of Table 1. The dotted line corresponds to the equation (2).

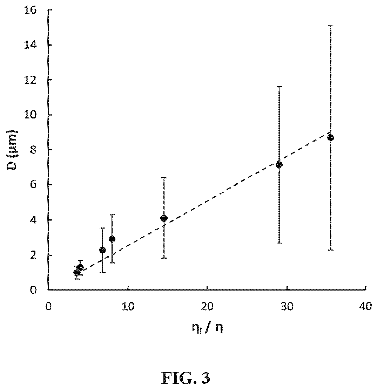

**Figure 3** is a graph showing the variation of the mean diameter (D) and size distribution of the formed droplets as a function of the ratio $\eta_i/\eta$ for the samples in Table 2. Data points correspond to the mean diameter (D) and error bars correspond to the standard deviation of the size distribution. The dotted line corresponds to equation (3).

## EXAMPLES

The present invention is further illustrated by the following examples.

All percentages mentioned for the compositions in the examples are weight percentages in respect to the total weight of the composition.

In all following examples, a dispersed phase composed of polyester polymer (CN2035, Sartomer) 92%, hexanediol diacrylate 5% and Darocur 1173 (Aldrich) 3% was used. This mixture is Newtonian with $\eta_i$ = 32000 mPa.s.

An emulsion made of 5% of this dispersed phase in various types of continuous phases was formed by applying a 500 s$^{-1}$ shear rate for one minute using a sawtooth impeller. Immediately after the shear rate was stopped, the formed droplets were cross-linked under UV light in order to freeze the system before ageing such as coalescence and growth could occur. It was therefore possible to measure the size distribution of the formed droplets as it is right after the application of shear, by means of image analysis of microscopy pictures of the cross-linked emulsions.

The monodispersity / polydispersity and size control of the formed droplets as a function of the continuous phase properties were observed and compared.

### Example 1: Droplets formation in viscoelastic media (0.2 < G"/G' < 10)

A variety of polymers solubilised in water were used as continuous phases, whose nature and properties are summarised in Table 1.

### *Results*

These polymer systems enable the formation of droplets with a mean diameter D ranging from 5 to 70 $\mu$m. These droplets are size-controlled as the mean diameter can be predicted from equation (1) mentioned above. The size distribution is found to be polydisperse when G"/G' is below 0.2 and monodisperse when G"/G' is above 0.2.

**Figure 1 (filled circles)** shows the variation of the mean diameter (D) and size distribution of the formed droplets as a function of G"/G' ratio at the working angular frequency for materials of Table 1. Data points correspond to the mean diameter (D) and error bars correspond to the standard deviation of the size distribution. The black dotted line corresponds to the equation (1).

Figure 1 shows that:

- If 0.2 < G"/G' < 1, a polydisperse population of size-controlled droplets is formed.
- If 1 < G"/G' < 10, a monodisperse population of size-controlled droplets is formed by an efficient elongation-fragmentation mechanism with a typical diameter below 20 $\mu$m.

Moreover, it was found that the ratio G"/G' varies with the product $\eta_{(1\%)}$.C verifying equation (2) mentioned above.

**Figure 2** shows the evolution of the G"/G' ratio as a function of the product $\eta_{(1\%)}$.C for the materials of Table 1. The grey dotted line corresponds to the equation (2).

Figure 2 shows that:

- If $\eta_{(1\%)}.C < 1\,000$ mPa.s, a monodisperse population of size-controlled droplets is formed and the continuous phase belongs to the predominant viscous regime.

- If $1\,000$ mPa.s $< \eta_{(1\%)}.C < 100\,000$ mPa.s, a polydisperse population of size-controlled droplets is formed.

**Table 1**

| Materials | Supplier | Chemistry | $\eta_{(1\%)}$ (mPa.s) | C (%) | $\eta_{(1\%)}.C$ | G''/G'$_{(\omega=100\text{ rad/s})}$ |
|---|---|---|---|---|---|---|
| Konjac 36000 | Roeper | Konjac | 90 000 | 1 | 90 000 | 0.23 |
| Carbomer 141 | Adara | Polyacrylic acid | 40 000 | 1.5 | 60 000 | 0.23 |
| CMC 4570 | Roeper | Carboxymethyl cellulose | 20 000 | 1,5 | 30 000 | 0.4 |
| CMC 4520 | Roeper | Carboxymethyl cellulose | 200 | 5 | 1000 | 0.78 |
| CMC 4500 100-200 | Roeper | Carboxymethyl cellulose | 60 | 8 | 480 | 0.92 |
| Polyox WSR 205 | Dow | Polyethylene oxyde | 70 | 5 | 350 | 1.12 |
| Chitosan | Chitosan lab | Chitosan | 20 | 5 | 100 | 1.13 |
| CMC 4500 20-50 | Roeper | Carboxymethyl cellulose | 20 | 13,5 | 270 | 1 . 13 |
| Chitosan | Chitosan lab | Chitosan | 33 | 10 | 330 | 1.15 |
| CMC 4520 | Roeper | Carboxymethyl cellulose | 200 | 3.4 | 680 | 1.16 |
| Methocel A4C | Dow | Methyl cellulose | 80 | 5 | 400 | 1.25 |
| Chitosan | Chitosan lab | Chitosan | 20 | 10 | 200 | 1.41 |

**Example 2: Droplet formation in Newtonian fluids (G''/G' > 10)**

A variety of polymers were used as continuous phases with Newtonian behavior (G''/G' > 10): hydrophobically modified ethoxylate-urethane copolymer (Optiflo T1000 and Optiflo L1400, Byk) and hydrophobically modified polyacetal-polyether copolymer (Aquaflow NHS300E, Ashland). The composition and viscosity of the different samples made are summarised in Table 2.

**Table 2**

| Composition | $\eta$ (mPa.s) |
|---|---|
| Aquaflow NHS300E 58% Water 42% | 900 |
| Optiflo T1000 70% Water 30% | 1 100 |
| Aquaflow NHS300E 75% Water 25% | 2 200 |
| Optiflo L1400 80% Water 20% | 4 000 |
| Optiflo L1400 85% Water 15% | 4 700 |
| Optiflo L1400 | 8 000 |
| Aquaflow NHS300E | 9 000 |

*Results*

If G''/G' > 10, the polymer solution behaves like a purely viscous Newtonian fluid. Thus, the mean diameter D does not depend on the G''/G' ratio and the size distribution remain broad.

All prepared continuous phases have a purely viscous behavior, and the diameter D of the formed droplets verifies equation (3), in which $\sigma$ is the surface tension, $\eta$ the viscosity of the continuous phase and $\gamma$ the shear rate. In particular, D does not depend on the G''/G' ratio:

$$(3)\ D \sim \sigma/(\eta\dot{\gamma})$$

The size distribution of the droplet population was found to be polydisperse.

**Figure 3** shows the variation of the mean diameter (D) and size distribution of the formed droplets as a function of the ratio $\eta_i/\eta$ for the samples in Table 2. Data points correspond to the mean diameter (D) and error bars correspond to the standard deviation of the size distribution. The dotted line corresponds to equation (3).

### Example 3: Insufficient fragmentation to form droplets (G"/G' < 0.2)

Two different polymers solubilised in water were used as continuous phases, whose nature and properties are summarised in Table 3.

**Table 3**

| Materials | Supplier | Chemistry | $\eta_{(1\%)}$ (mPa.s) | C (%) | $\eta_{(1\%)}$.C | G"/G'$_{(\omega=100\ rad/s)}$ |
|---|---|---|---|---|---|---|
| Guar 3500 | Roeper | Guar | 20 000 | 5 | 100 000 | 0.15 |
| CMC 4570 | Roeper | Carboxymethyl cellulose | 20 000 | 6 | 120 000 | 0.18 |

*Results*

In these systems wherein G"/G' < 0.2 and $\eta_{(1\%)}$.C > 100 000 mPa.s no droplets could be formed, showing that the elastic modulus is too high for efficient fragmentation.

### Example 4: Addition of a Newtonian fluid in the continuous phase

Different mixtures of a chitosan (Chitosanlab) solution at 5% in water and Optiflo L1400 (Byk) were used as continuous phases. Their composition and properties are summarised in Table 4.

*Results*

These polymer systems enable the formation of droplets with a mean diameter D below 10 $\mu$m. These droplets are size-controlled as the mean diameter can be predicted from equation (1) mentioned above. The size distribution is found to be monodisperse which is consistent with the fact that G"/G' is above 0.2. These results can be visualised in **Figure 1** (open squares).

Moreover, it is found that G"/G' increases linearly with the concentration of the Newtonian fluid.

**Table 4**

| Materials | Chitosan 5% Conc. (%) | Optiflo L1400 Conc. (%) | G"/G'$_{(\omega=100\ rad/s)}$ |
|---|---|---|---|
| 1 | 100 | 0 | 1.13 |
| 2 | 97,5 | 2,5 | 1.2 |
| 3 | 95 | 5 | 1.28 |
| 4 | 90 | 10 | 1.36 |
| 5 | 80 | 20 | 1.43 |
| 6 | 60 | 40 | 1.58 |

### Claims

1. Emulsion comprising droplets dispersed in a viscoelastic continuous phase,
   said droplets having a mean diameter inferior to 100 $\mu$m,
   wherein said droplets are size-controlled,
   said viscoelastic continuous phase comprising at least one polymer in solution in at least one solvent,

wherein said at least one polymer is selected from cellulose derivatives, polyacrylates, polyacrylamides, polysaccharides, protein derivatives, silicone derivatives and a mixture thereof,

provided that said at least one polymer is not an alginate,

wherein said viscoelastic continuous phase present a ratio between the viscous modulus **(G")** and the elastic modulus **(G')** ranging from 0.2 to 10; and

wherein the mean diameter of said droplets is proportional to the **G'/G"** ratio with 1.5 as exponent.

2. The emulsion according to claim **1,** wherein said viscoelastic continuous phase does not comprise a surfactant.

3. The emulsion according to claim **1** or claim **2,** wherein said viscoelastic continuous phase comprises exactly one polymer in solution in at least one solvent;

wherein the product $\eta_{(1\%)}.\textbf{C}$ ranges from 100 mPa.s to 100 000 mPa.s, preferably from 100 mPa.s to 1000 mPa.s; wherein $\eta_{(1\%)}$ represents the viscosity of the polymer solution at 1% concentration by weight in respect to the total weight of the viscoelastic continuous phase and **C** represents the concentration of the polymer solution; and wherein the **G"/G'** ratio is proportional to the product $\eta_{(1\%)}.\textbf{C}$ with -0.3 as exponent.

4. The emulsion according to any one of claims **1** to **3,** wherein said at least one polymer is in solution in at least one solvent at a concentration ranging from 1 to 20 % by weight in respect to the total weight of the viscoelastic continuous phase.

5. The emulsion according to any one of claims **1** to **4,** wherein said polymer is selected from chitosan, carboxymethyl cellulose, methyl cellulose, guar, konjac, polyacrylic acid, polyethylene oxide and a mixture thereof.

6. The emulsion according to any one of claims **1** to **5,** wherein said droplets are size-controlled monodisperse droplets, and wherein said **G"/G'** ratio is ranging from 1 to 5, preferably said **G"/G'** ratio is ranging from 1 to 3.

7. The emulsion according to any one of claims **1** to **6,** wherein said droplets have a mean diameter ranging from 1 to 70 $\mu$m, preferably ranging from 5 to 20 $\mu$m.

8. The emulsion according to any one of claims **1** to **7,** wherein said viscoelastic continuous phase further comprises at least one Newtonian fluid.

9. The emulsion according to any one of claims **1** to **8,** wherein the dispersed phase comprises at least one crosslinkable material and optionally at least one initiator.

10. The emulsion according to claim **9,** wherein the dispersed phase further comprises at least one non-miscible active agent.

11. Process for the manufacture of an emulsion according to any one of claims **1** to **10,** comprising a step of submitting the dispersed droplets to an elongation-fragmentation mechanism in the viscoelastic continuous phase.

12. The process according to claim **11,** wherein a shear rate ranging from 1 to 20000 s$^{-1}$ is applied during the elongation-fragmentation step, preferably from 1 to 1000 s$^{-1}$.

13. The process according to claim **11** or claim **12,** further comprising a step of addition of a Newtonian fluid into the viscoelastic continuous phase.

14. Process for the fabrication of microcapsules comprising the steps of:

- manufacturing an emulsion as described in any one of claims **11** to **13,** and
- cross-linking the formed droplets.

15. The process according to claim **14,** wherein the cross-linking of the formed droplets is performed by photopolymerization of said emulsion.

**FIG. 1**

**FIG. 2**

**FIG. 3**

Europäisches
Patentamt

European
Patent Office

Office européen
des brevets

## EUROPEAN SEARCH REPORT

Application Number

EP 20 30 6476

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHEKARFOROUSH ELHAMALSADAT ET AL: "Soy Protein-Gum Karaya Conjugate: Emulsifying Activity and Rheological Behavior in Aqueous System and Oil in Water Emulsion", JOURNAL OF THE AMERICAN OIL CHEMISTS SOCIETY, SPRINGER, DE, vol. 93, no. 1, 16 November 2015 (2015-11-16), pages 1-10, XP035947522, ISSN: 0003-021X, DOI: 10.1007/S11746-015-2751-Z [retrieved on 2015-11-16] * abstract * * page 3, right-hand column, paragraph 1 * * page 5; table 2 * * page 8; figure 5 * ----- | 1-3,7, 11,12 | INV. A61K9/107 A61K47/32 A61K47/36 A61K47/38 A61K47/34 A61K8/06 |
| X | MANOJ PRETIMA ET AL: "Characterization of a Polydisperse Depletion-Flocculated Emulsion", JOURNAL OF COLLOID AND INTERFACE SCIENCE, vol. 228, no. 2, August 2000 (2000-08), pages 200-206, XP055803685, US ISSN: 0021-9797, DOI: 10.1006/jcis.2000.6936 * abstract * * page 201, left-hand column, paragraph 1-4; figure 1 * * page 202; figure 3 * * page 204; figure 7 * ----- | 1,3,6,7, 11,12 | |

TECHNICAL FIELDS SEARCHED (IPC)

A61K
A61Q

-/--

The present search report has been drawn up for all claims

| Place of search | Date of completion of the search | Examiner |
|---|---|---|
| Munich | 14 May 2021 | Nyeki, Agnes |

EPO FORM 1503 03.82 (P04C01)

Europäisches
Patentamt

European
Patent Office

Office européen
des brevets

**EUROPEAN SEARCH REPORT**

Application Number

EP 20 30 6476

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NI XUEWEN ET AL: "Physical stability and rheological properties of konjac glucomannan-ethyl cellulose mixed emulsions", INTERNATIONAL JOURNAL OF BIOLOGICAL MACROMOLECULES, ELSEVIER BV, NL, vol. 92, 5 July 2016 (2016-07-05), pages 423-430, XP029755256, ISSN: 0141-8130, DOI: 10.1016/J.IJBIOMAC.2016.07.018 * the whole document * * page 428; figures 6C, 6D * ----- | 1,3,5-7, 11,12 | |
| X | US 2008/071077 A1 (DIJK BEREND J [NL] ET AL) 20 March 2008 (2008-03-20) * page 1, paragraphs 14,15 * * page 3, paragraph 41 - page 4, paragraph 42; example 4 * * claims 1-5 * * figure 2 * ----- | 1,3-5,7, 10-12 | |
| X | US 2020/094214 A1 (WALTERS JAMIE [FR] ET AL) 26 March 2020 (2020-03-26) * the whole document * * pages 7-9; examples 1-3 * ----- | 1-15 | TECHNICAL FIELDS SEARCHED (IPC) |

The present search report has been drawn up for all claims

| Place of search | Date of completion of the search | Examiner |
|---|---|---|
| Munich | 14 May 2021 | Nyeki, Agnes |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 008 312 A1**

## ANNEX TO THE EUROPEAN SEARCH REPORT
## ON EUROPEAN PATENT APPLICATION NO.

EP 20 30 6476

14-05-2021

| Patent document cited in search report | | Publication date | Patent family member(s) | | Publication date |
|---|---|---|---|---|---|
| US 2008071077 | A1 | 20-03-2008 | NONE | | |
| US 2020094214 | A1 | 26-03-2020 | AU | 2017367182 A1 | 20-06-2019 |
| | | | BR | 112019011248 A2 | 15-10-2019 |
| | | | CA | 3045215 A1 | 07-06-2018 |
| | | | CN | 110062779 A | 26-07-2019 |
| | | | EP | 3548529 A1 | 09-10-2019 |
| | | | FR | 3059666 A1 | 08-06-2018 |
| | | | JP | 2020500707 A | 16-01-2020 |
| | | | KR | 20190126048 A | 08-11-2019 |
| | | | US | 2019388861 A1 | 26-12-2019 |
| | | | US | 2020094214 A1 | 26-03-2020 |
| | | | WO | 2018100179 A1 | 07-06-2018 |

EPO FORM P0459

For more details about this annex : see Official Journal of the European Patent Office, No. 12/82

**REFERENCES CITED IN THE DESCRIPTION**

**Patent documents cited in the description**

- WO 9738787 A